# EUROPÄISCHE PATENTSCHRIFT

(11) **EP 2 624 872 B1**
(45) Veröffentlichungstag und Bekanntmachung des Hinweises auf die Patenterteilung: **27.02.2019**
(21) Anmeldenummer: 11763706.6
(22) Anmeldetag: 30.09.2011
(51) Int. Cl.: A61L 27/30, A61L 27/50, A61F 2/36

(54) **PROTHESENKOMPONENTE MIT ANTIMIKROBIELL BESCHICHTETER GLEITFLÄCHE**
PROSTHESIS COMPONENT WITH ANTIMICROBIALLY COATED SLIDE SURFACE
COMPOSANT DE PROTHÈSE AYANT UNE SURFACE DE GLISSEMENT À REVÊTEMENT ANTIMICROBIEN

(30) Priorität: 05.10.2010 EP 10013320
(43) Veröffentlichungstag der Anmeldung: 14.08.2013
(73) Patentinhaber: Waldemar Link GmbH & Co. KG, 22339 Hamburg (DE)
(72) Erfinder: THULL, Roger, 97082 Würzburg (DE)
(74) Vertreter: Hoffmann Eitle
(86) Internationale Anmeldenummer: PCT/EP2011/067072
(87) Internationale Veröffentlichungsnummer: WO 2012/045672

(56) Entgegenhaltungen:
- EP-A1- 2 036 517
- WO-A2-2007/084961
- DE-A1-102008 008 517
- US-A1- 2009 198 343

## Beschreibung

Die Erfindung betrifft eine Prothesenkomponente einer Gelenkendoprothese. Die Prothesenkomponente umfasst eine Gleitfläche, die dazu ausgelegt ist, mit einer Gegengleitfläche ein Gleitgelenk zu bilden. Die Gegengleitfläche kann eine Fläche einer anderen Prothesenkomponente sein oder ein knöchernes Gegenlager des Gelenks bzw. Weichgewebe.

Wenn Prothesen in den menschlichen Körper eingesetzt werden, ergeben sich immer wieder Schwierigkeiten, weil der Körper die Prothese nicht komplikationslos annimmt. Häufig sind sogenannte Biofilme, die sich nach Besiedlung der Oberfläche mit Bakterien auch im implantierten Zustand bilden, die Ursache für Komplikationen. Es hat sich gezeigt, dass ein Risiko nicht nur von den Oberflächenbereichen der Prothese ausgeht, die im implantierten Zustand direkt an Körpergewebe anliegen, sondern auch von solchen Oberflächenbereichen, die dazu bestimmt sind, mit anderen Prothesenkomponenten zusammenzuwirken. Zu diesen Oberflächenbereichen gehören insbesondere die Gleitflächen einer Prothesenkomponente, die mit anderen Prothesenkomponenten zusammenwirken, um die Funktionalität des natürlichen Gelenks nachzubilden. Oberflächenbiofilme in diesen Bereichen sind den Abwehrmechanismen des Körpers weitgehend entzogen. Es besteht die Gefahr, dass Biofilme aus dem Bereich der Gleitflächen heraus immer wieder Mikroorganismen in die Umgebung freisetzen. Die Neigung der Mikroorganismen, sich aus dem Bereich der Gleitflächen heraus in die Umgebung zu verteilen, wird noch dadurch verstärkt, dass die Gleitflächen laufend Bewegungen ausgesetzt sind.
Die Gleitflächen der Prothesen sind ganz anderen Belastungen ausgesetzt als die übrigen Bereiche der Oberfläche. Gegebenenfalls finden Relativbewegungen in dem Gelenk statt, während nahezu das gesamte Gewicht des menschlichen Körpers vermehrt um Beschleunigungskräfte in Funktion auf den Gleitflächen der Prothese lasten. Es ist unter diesen Bedingungen schwierig, die Ausbreitung der Mikroorganismen zu verhindern. US 2009/198343 A1 offenbart eine Prothesenkomponente einer Gelenkendoprothese mit einer Gleitfläche, wobei die Gleitflächenbeschichtung Silber und Chromnitrid enthält. Ausgehend von diesem Stand der Technik liegt der Erfindung die Aufgabe zugrunde, eine Prothesenkomponente vorzustellen, bei der die Wahrscheinlichkeit vermindert ist, dass es bei der Aufnahme in den menschlichen Körper zu Komplikationen kommt. Die Aufgabe wird gelöst durch die Merkmale des Anspruch 1, das heißt durch eine Prothesenkomponente einer Gelenkprothese mit einer Gleitfläche (19, 24), die dazu ausgelegt ist, mit einer Gegengleitfläche (26) einer anderen Prothesenkomponente (11, 23) oder dem knöchernen Gegenlager des Gelenks bzw. Weichgewebe ein Gleitgelenk zu bilden, wobei die Gleitfläche (19, 24) von einer auf den Körper der Prothesenkomponente (10, 22) aufgebrachten Gleitflächenbeschichtung (20) gebildet ist und die Gleitflächenbeschichtung (20) eine antimikrobielle Wirkung hat; wobei in der Gleitflächenbeschichtung (20) Silber enthalten ist und die Gleitflächenbeschichtung (20) ein Nitrid, ein Oxinitrid oder ein Oxid auf der Basis eines Refraktärmetalls umfasst, wobei das Silber (21) einen Gewichtsanteil zwischen 2% und 15%, vorzugsweise zwischen 3% und 10% in der Gleitflächenbeschichtung (20) bildet, dadurch gekennzeichnet, dass das Refraktärmetall Titan ist.

Erfindungsgemäß ist die Prothesenkomponente also mit einer Gleitflächenbeschichtung versehen, die eine antimikrobielle Wirksamkeit hat. Vorteilhafte Ausführungsformen finden sich in den Unteransprüchen.

Zunächst werden einige Begriffe erläutert. Eine Beschichtung mit antimikrobieller Wirksamkeit zeichnet sich dadurch aus, dass sie das Potenzial hat, die Lebensfähigkeit oder die Vermehrungsfähigkeit von Mikroorganismen zu vermindern. Regelmäßig wird dies dadurch erreicht, dass aus der Beschichtung heraus eine Substanz abgegeben wird, die eine Wirksamkeit gegen Mikroorganismen hat.

Das Gleitgelenk einer Prothese wird gebildet durch die Gleitfläche einer ersten Prothesenkomponente und eine zugehörige Gegengleitfläche einer zweiten Prothesenkomponente. Während eines Bewegungsablaufs kann der Kontakt zwischen Gleitfläche und Gegengleitfläche in wechselnden Flächenbereichen der Gleitfläche bestehen. Der Begriff Gleitfläche umfasst alle Flächenbereiche, die bei Gelenkbewegungen in der Prothese mit der Gegengleitfläche in Kontakt kommen können.

Wenn eine Beschichtung auf einen Körper aufgebracht wird, wird dem Körper zusätzliches Material hinzugefügt. Auf der Oberfläche des Körpers entsteht eine Schicht, die vollständig aus neu hinzugefügtem Material besteht. Nicht ausgeschlossen ist es, dass die erfindungsgemäße Beschichtung auch auf anderen Oberflächenbereichen der Prothesenkomponente aufgebracht sein kann als auf der Gleitfläche. Häufig sind aber die anderen Oberflächenbereiche frei von der Beschichtung, so dass die Beschichtung nur auf der Gleitfläche ist.

Mit der erfindungsgemäßen Beschichtung wird die antimikrobielle Wirkung in den Bereich der Gleitflächen hineingebracht und damit in einen Bereich, der für die körpereigenen Abwehrkräfte nicht zugänglich ist. Ohne antimikrobielle Wirkung im Bereich der Gleitflächen könnten sich dort Mikroorganismen festsetzen und immer wieder vermehren. Die erfindungsgemäße antimikrobielle Beschichtung wirkt gegen die Mikroorganismen und vermindert damit das Risiko von Komplikationen.

Antimikrobielle Beschichtungen als solche auf der Oberfläche von Prothesen sind bekannt, siehe etwa EP 2 036 517. Sie waren aber bislang nur für solche Oberflächenbereiche der Prothese vorgesehen, die geringen mechanischen Belastungen ausgesetzt sind.

Ein für den Einsatz im Körper besonders geeigneter antimikrobieller Wirkstoff ist Silber. Silberpartikel, die aus einer Beschichtung heraus abgegeben werden, haben eine gute Wirksamkeit gegen Mikroorganismen, die auf der Oberfläche der Beschichtung sitzen. Treten die Silberpartikel aus der Beschichtung aus, ohne auf Mikroorganismen zu treffen, kombinieren sie im Körperelektrolyt zu Silberchlorid (AgCl) und können in dieser Form aus dem Körper ausgeschieden werden. Das Silber hat dann keine schädigende Wirkung auf andere Körperzellen. Die Gleitflächenbeschichtung der erfindungsgemäßen Prothesenkomponente umfasst deswegen Silber. Das Silber wird aus der Gleitflächenbeschichtung heraus vorzugsweise in Form von einzelnen Silberionen abgegeben.
Die Gleitflächen einer Gelenkprothese sind hohen mechanischen Belastungen ausgesetzt. Eine große Belastbarkeit der Gleitflächen kann erreicht werden, indem die Gleitflächenbeschichtung eine Hartstoffbeschichtung ist. Erfindungsgemäß umfasst die Gleitflächenbeschichtung ein Nitrid, ein Oxinitrid oder ein Oxid auf der Basis von Titan als Refraktärmetall. Erfindungsgemäß ist in der Gleitflächenbeschichtung außerdem Silber enthalten, das aus der Beschichtung heraus abgegeben werden kann. Refraktärmetalle sind hochschmelzende, unedle Metalle der vierten, fünften und sechsten Nebengruppe. Umfasst sind in der vierten Nebengruppe Titan, Zirconium und Hafnium, in der fünften Nebengruppe Vanadium, Niob und Tantal sowie in der sechsten Nebengruppe Chrom, Molybdän und Wolfram.

Als Nitrid, Oxinitrid oder Oxid auf der Basis eines Refraktärmetalls werden Verbindungen bezeichnet, die die Ionen eines Refraktärmetalls mit Sauerstoff und/oder Stickstoff als reaktivem Gas eingehen. Diese Verbindungen zeichnen sich durch eine große Härte aus. Wenn die Gleitflächenbeschichtung zusätzlich Silber umfasst, so sind bei der Entstehung des Nitrids, Oxinitrids bzw. Oxids nicht nur Ionen des Refraktärmetalls und ein reaktives Gas, sondern zusätzlich Silberionen beteiligt. Die Silberionen werden in die entstehende Beschichtung integriert.

Bei einer Hartstoffbeschichtung, die Silber enthält, steigt die antimikrobielle Wirksamkeit mit dem Silbergehalt. Würde man die Beschichtung in erster Linie auf eine hohe antimikrobielle Wirksamkeit auslegen, würde man einen Gewichtsanteil von etwa 25% Silber in der Hartstoffbeschichtung wählen. Allerdings ist die mechanische Belastbarkeit im Vergleich mit einer Hartstoffbeschichtung, die kein Silber enthält, dann deutlich vermindert. Im Rahmen der Erfindung sollte deswegen ein geringerer Gewichtsanteil Silber in der Hartstoffbeschichtung gewählt werden. Der Gewichtsanteil liegt erfindungsgemäß zwischen 2% und 15%, vorzugsweise zwischen 3% und 10%. Es hat sich gezeigt, dass mit einem derart geringen Silbergehalt ein guter Kompromiss zwischen mechanischer Belastbarkeit und antimikrobieller Wirkung erzielt wird.

Die Gleitflächenbeschichtung sollte so zusammengesetzt sein, dass das Silber in erster Linie in Form einzelner Silberionen und nicht in Form größerer Partikel abgegeben wird. Einzelne Silberionen können zwischen den Gleitflächen wirken, ohne dass sie eine abrasive Wirkung gegenüber den Gleitflächen entfalten.

Der Körper der Prothesenkomponente kann aus Metall oder aus einer Metalllegierung bestehen. Besonders geeignete Materialien sind Titan und Titanlegierungen.

Eine erfindungsgemäße Gleitflächenbeschichtung kann beispielsweise mittels eines PVD-Verfahrens (Physical Vapor Deposition) auf den Körper der Prothesenkomponente aufgebracht werden. Es werden dazu Targets bereitgestellt, aus denen das Material, das später die Beschichtung bilden soll, herausgelöst werden kann. Es kann ein einzelnes Target bereitgestellt werden, das sowohl das Refraktärmetall als auch das Silber umfasst. Möglich ist es aber auch, mehrere Targets bereitzustellen, wobei ein erstes Target ein Refraktärmetall umfasst und wobei ein zweites Target Silber umfasst.

Zum Herauslösen der Ionen kann beispielsweise ein Lichtbogen zwischen einer Elektrode und dem Target erzeugt werden, der dem Target lokal begrenzt soviel Energie zuführt, dass Ionen herausgelöst werden. Eine andere Möglichkeit, dem Target lokal ausreichend Energie zuzuführen, kann darin bestehen, einen Laserstrahl auf das Target zu richten. Alternativ, wenn auch sehr teuer, ist die Energiezufuhr auch durch einen Elektronenstrahl möglich.

Die freien Ionen werden auf den Körper der Prothesenkomponente geleitet. Dazu kann eine Spannung zwischen das Target und den Körper gelegt werden, durch die die Ionen in Richtung des Körpers beschleunigt werden. Das Reaktionsgefäß mit Target und Körper enthält ein reaktives Gas, wie beispielsweise Sauerstoff oder Stickstoff oder ein Gemisch von Sauerstoff und Stickstoff. Die Ionen des Refraktärmetalls sowie die Ionen von Silber und/oder Kupfer reagieren mit dem reaktiven Gas. Durch die auf der Oberfläche des Körpers stattfindende Reaktion bildet sich die erfindungsgemäße Gleitflächenbeschichtung. Die Reaktion kann unter Vakuum, vorzugsweise unter Hochvakuum stattfinden.

Wenn verhindert werden soll, dass die Beschichtung sich auch auf anderen Oberflächenbereichen der Prothesenkomponente als auf der Gleitfläche aufbaut, können diese Oberflächenbereiche während des Beschichtens abgedeckt werden. Beispielsweise können diese Oberflächenbereiche mit einem temperaturstabilen Material belegt werden. Als temperaturstabil wird ein Material bezeichnet, das gegenüber den bei Plasma-Beschichtungsverfahren bestehenden Bedingungen beständig ist. Das temperaturstabile Material kann insbesondere ein Metall sein.

Um den Abrieb bei Bewegungen zwischen der Gleitfläche und der Gegengleitfläche gering zu halten, sollte die Gleitfläche möglichst glatt sein. Vorzugsweise ist die nach DIN EN ISO 4288 und 3274 bestimmte mittlere Rauigkeit Rₐ der Gleitfläche kleiner 0,05 µm.

Die Erfindung betrifft außerdem eine Gelenkendoprothese mit einer erfindungsgemäß augebildeten ersten Prothesenkomponente und einer zweiten Prothesenkomponente. Die zweite Prothesenkomponente umfasst eine Gegengleitfläche, die mit der Gleitfläche der ersten Prothesenkomponente ein Gleitgelenk bildet.

Die Erfindung wird nachfolgend unter Bezugnahme auf die beigefügten Zeichnungen anhand einer vorteilhaften Ausführungsform beispielhaft beschrieben. Es zeigen:
- Fig. 1: eine erfindungsgemäß ausgerüstete Hüftprothese;
- Fig. 2: einen vergrößerten Ausschnitt aus Fig. 1; und
- Fig. 3: eine erfindungsgemäß ausgerüstete Knieprothese.

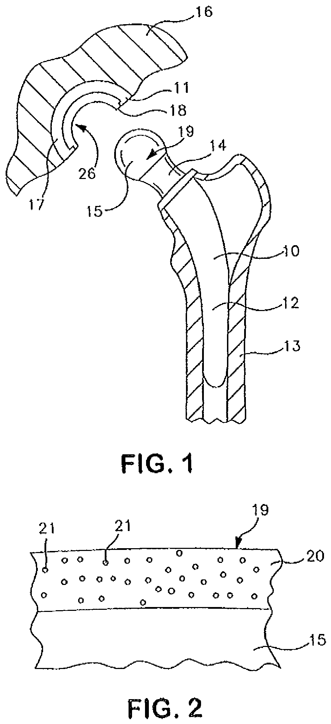

Eine in Fig. 1 gezeigte Hüftprothese umfasst eine Femurkomponente 10 und eine Acetabulumkomponente 11. Die Femurkomponente 10 umfasst einen Schaft 12, der in den Markraum eines Oberschenkelknochens 13 eingesetzt ist. An den Schaft 12 schließt sich ein Prothesenhals 14 an, der in einen Gelenkkopf 15 übergeht. Die Acetabulumkomponente 11 ist in den Hüftknochen 16 eingebracht und ersetzt dort die natürliche Hüftpfanne. Die Acetabulumkomponente 11 umfasst eine aus Metall bestehende Schale 17 sowie einen Einsatz 18, der aus hochverdichtetem Polyethylen besteht.

Im implantierten Zustand liegt der Gelenkkopf 15 in dem Polyethylen-Einsatz 18, so dass der Gelenkkopf 15 zusammen mit dem Polyethylen-Einsatz 18 ein Gleitgelenk bildet. Dabei wirkt die Oberfläche des Gelenkkopfs 15 als Gleitfläche 19 mit einer Gegengleitfläche 26 des Polyethylen-Einsatzes 18 zusammen. Die mittlere Rauigkeit Rₐ der Gleitfläche ist kleiner als 0,03 µm.

Die Femurkomponente 10 ist aus einer Titanlegierung gefertigt. Im Bereich der Gleitfläche 19 ist, wie Fig. 2 in stark vergrößerter Darstellung zeigt, eine Gleitflächenbeschichtung 20 auf den Gelenkkopf 15 aufgebracht. Die Gleitflächenbeschichtung 20 ist mittels eines PVD-Verfahrens hergestellt und hat eine Dicke von ungefähr 3 µm. Die Gleitflächenbeschichtung 20 besteht aus Titannitrid mit eingeschlossenen Silberatomen 21, wobei die Silberatome nicht maßstabsgerecht dargestellt sind. In der Gleitflächenbeschichtung 20 machen die Silberatome einen Gewichtsanteil von ungefähr 5% aus.

Das Silber ist in Form einzelner Atome und nicht in Form größerer Partikel in der Gleitflächenbeschichtung 20 enthalten. Bei der Benutzung der Prothese lösen sich einzelne Silberionen aus der Gleitflächenbeschichtung 20 heraus. Die Silberionen üben im Bereich des Gleitgelenks eine antimikrobielle Wirkung aus. Die einzelnen Silberionen sind so klein, dass sie keine abrasive Wirkung auf den Polyethylen-Einsatz 18 ausüben.

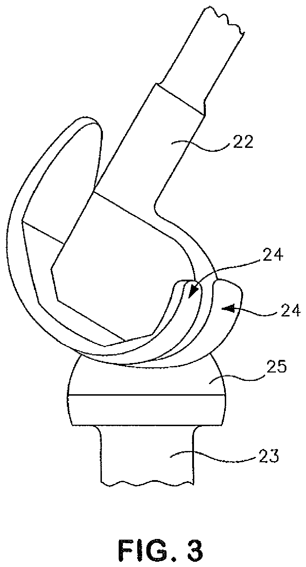

In Fig. 3 ist eine Knieprothese gezeigt, die mit einer erfindungsgemäßen Gleitflächenbeschichtung ausgerüstet ist. Die Knieprothese umfasst eine Femurkomponente 22 und eine Tibiakomponente 23. Gleitflächen 24 der Femurkomponente 22 wirken mit einem Polyethylen-Einsatz 25 der Tibiakomponente 23 zusammen und bilden ein Gleitgelenk, das die Funktion des natürlichen Knies nachbildet.

Die Femurkomponente 22 und die Tibiakomponente 23 bestehen aus einer klassischen Chrom-Kobalt-Legierung. Auf die Gleitflächen 24 der Femurkomponente 22 ist eine antimikrobielle Gleitflächenbeschichtung 20 aufgebracht, wie sie in Fig. 2 dargestellt ist.

Bei der Knieprothese der Fig. 3 liegt zu jedem Zeitpunkt nur ein kleiner Flächenbereich der Gleitflächen 24 auf dem Polyethylen-Einsatz 25 auf. Der entsprechende Flächenbereich ändert sich je nach dem Bewegungszustand der Knieprothese. Die Gleitflächenbeschichtung 20 erstreckt sich über die gesamten Gleitflächen 24, also über alle Flächenbereiche, die mit dem Polyethylen-Einsatz 25 in Berührung kommen können. Die aus der Gleitflächenbeschichtung 20 austretenden Silberionen haben eine antimikrobielle Wirkung gegen dort sitzende Mikroorganismen.

## Patentansprüche

1. Prothesenkomponente einer Gelenkendoprothese mit einer Gleitfläche (19, 24), die dazu ausgelegt ist, mit einer Gegengleitfläche (26) einer anderen Prothesenkomponente (11, 23) oder dem knöchernen Gegenlager des Gelenks bzw. Weichgewebe ein Gleitgelenk zu bilden, wobei die Gleitfläche (19, 24) von einer auf den Körper der Prothesenkomponente (10, 22) aufgebrachten Gleitflächenbeschichtung (20) gebildet ist und die Gleitflächenbeschichtung (20) eine antimikrobielle Wirkung hat; wobei in der Gleitflächenbeschichtung (20) Silber enthalten ist und die Gleitflächenbeschichtung (20) ein Nitrid, ein Oxinitrid oder ein Oxid auf der Basis eines Refraktärmetalls umfasst, wobei das Silber (21) einen Gewichtsanteil zwischen 2% und 15%, vorzugsweise zwischen 3% und 10% in der Gleitflächenbeschichtung (20) bildet, **dadurch gekennzeichnet, dass** das Refraktärmetall Titan ist.

2. Prothesenkomponente nach Anspruch 1, **dadurch gekennzeichnet, dass** die Gleitflächenbeschichtung (20) eine Dicke zwischen 0,5 µm und 5 µm hat.

3. Prothesenkomponente nach einem der Ansprüche 1 bis 2, **dadurch gekennzeichnet, dass** die Gleitflächenbeschichtung (20) durch ein PVD-Verfahren erhältlich ist.

4. Prothesenkomponente nach einem der Ansprüche 1 bis 3, **dadurch gekennzeichnet, dass** die mittlere Rauigkeit Rₐ der Gleitfläche (19, 24) kleiner ist als 0,05 µm.

5. Gelenkendoprothese mit einer ersten Prothesenkomponente, die nach einem der Ansprüche 1 bis 4 ausgebildet ist, und mit einer zweiten Prothesenkomponente (11, 23), die eine Gegengleitfläche (26) aufweist, wobei die Gleitfläche (19, 24) der ersten Prothesenkomponente mit der Gegengleitfläche (26) der zweiten Prothesenkomponente (11, 23) ein Gleitgelenk bildet.

## Claims

1. Prosthesis component of a joint endoprosthesis, with a sliding surface (19, 24) which is designed to form a sliding joint together with a counterpart sliding surface (26) of another prosthesis component (11, 23) or with the osseous counterpart bearing of the joint or soft tissue, wherein the sliding surface (19, 24) is formed by a sliding surface coating (20) applied to the body of the prosthesis component (10, 22), and the sliding surface coating (20) has an antimicrobial action, wherein the sliding surface coating (20) contains silver, and the sliding surface coating (20) comprises a nitride, an oxinitride or an oxide based on a refractory metal, wherein the silver (21) has a percentage by weight of between 2% and 15%, preferably of between 3% and 10%, in the sliding surface coating (20), **characterized in that** the refractory metal is titanium.

2. Prosthesis component according to Claim 1, **characterized in that** the sliding surface coating (20) has a thickness between 0.5 µm and 5 µm.

3. Prosthesis component according to either of Claims 1 and 2, **characterized in that** the sliding surface coating (20) is obtainable by a PVD process.

4. Prosthesis component according to one of Claims 1 to 3, **characterized in that** the mean roughness Rₐ of the sliding surface (19, 24) is less than 0.05 µm.

5. Joint endoprosthesis with a first prosthesis component, which is configured according to one of Claims 1 to 4, and with a second prosthesis component (11, 23), which has a counterpart sliding surface (26), wherein the sliding surface (19, 24) of the first prosthesis component forms a sliding joint together with the counterpart sliding surface (26) of the second prosthesis component (11, 23).

## Revendications

1. Composant d'une prothèse d'articulation comprenant une surface de glissement (19, 24) qui est adaptée pour former une articulation à glissement avec une surface de glissement homologue (26) d'un autre composant de prothèse (11, 23) ou le contre-appui osseux de l'articulation ou du tissu mou, la surface de glissement (19, 24) étant formée par un revêtement de surface de glissement (20) appliquée sur le corps du composant (10, 22) de la prothèse et le revêtement de surface de glissement (20) ayant une action antimicrobienne ; le revêtement de surface de glissement (20) contenant de l'argent, et le revêtement de surface de glissement (20) contenant un nitrure, un oxynitrure ou un oxyde à base d'un métal réfractaire, l'argent (21) constituant une proportion en poids comprise entre 2 % et 15 %, de préférence entre 3 % et 10 %, du revêtement de surface de glissement (20), **caractérisé en ce que** le métal réfractaire est du titane.

2. Composant de prothèse selon la revendication 1, **caractérisé en ce que** le revêtement de surface de glissement (20) a une épaisseur comprise entre 0,5 µm et 5 µm.

3. Composant de prothèse selon l'une des revendications 1 à 2, **caractérisé en ce que** le revêtement de surface de glissement (20) peut être obtenu par un procédé PVD.

4. composant de prothèse selon l'une des revendications 1 à 3, **caractérisé en ce que** la rugosité moyenne Rₐ de la surface de glissement (19, 24) est inférieure à 0,05 µm.

5. Endoprothèse d'articulation comprenant un premier composant de prothèse qui est conçu selon l'une des revendications 1 à 4, et un deuxième composant de prothèse (11, 23) qui comporte une surface de glissement homologue (26), la surface de glissement (19, 24) s du premier composant de prothèse formant une articulation à glissement avec la surface de glissement homologue (26) du deuxième composant de prothèse (11, 23) .
